# EUROPÄISCHE PATENTSCHRIFT

(11) **EP 2 007 458 B1**
(45) Veröffentlichungstag und Bekanntmachung des Hinweises auf die Patenterteilung: **02.05.2018**
(21) Anmeldenummer: 07727804.2
(22) Anmeldetag: 04.04.2007
(51) Int. Cl.: A61M 15/00

(54) **INHALATOR**
INHALER
INHALATEUR

(30) Priorität: 11.04.2006 DE 102006016903
(43) Veröffentlichungstag der Anmeldung: 31.12.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: BOECK, Georg, 88471 Laupheim (DE)
(74) Vertreter: Simon, Elke Anna Maria
(86) Internationale Anmeldenummer: PCT/EP2007/053335
(87) Internationale Veröffentlichungsnummer: WO 2007/116002

(56) Entgegenhaltungen:
- EP-A1- 0 388 621
- EP-A1- 1 238 680
- WO-A-2006/090149
- US-A- 5 152 284

## Beschreibung

Die Erfindung bezieht sich auf einen Inhalator nach Anspruch 1 zur Verabreichung von einem Arzneimittel in Form von inhalationsfähigen Substanzen, Substanzformulierungen oder -mischungen mit einem einen Innenraum zur Aufnahme der Substanzen aufweisenden Gehäuse, das mit einem Mundstück gekoppelt ist
Die EP 0 911 047 A1 offenbart einen Inhalator für die Inhalation pulverförmiger Arzneimittel aus Kapseln, der ein Unterteil mit zwei Fenstern und eine Platte, in der sich Kapselhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Kopf vorgesehen ist. Ein Mundrohr ist mit einem Oberteil des Inhalators und ein Deckel klappbar mit dem Unterteil, der Platte sowie dem Oberteil verbunden. EP 0 388 621 offenbart die bekannten Merkmale des Anspruchs 1. Um das Arzneimittel wirkungsvoll zu inhalieren, muss der Patient das Mundstück des Inhalators mit den Mundschleimhäuten (Lippen, Mund/Rachenraum) in Verbindung bringen. Dies erweist sich insofern als problematisch, als die Mundschleimhäute bei allen Menschen eine unterschiedlich große Anzahl verschiedenster Bakterien sowie andere Mikroorganismen aufweisen, die gegebenenfalls Krankheitserreger sein können. Demnach wird das Mundstück des Inhalators beim Gebrauch kontaminiert. Die Patienten und damit die Benutzer von Inhalatoren sind zur Reinigung des Mundstücks nach Gebrauch des Inhalators angehalten, wobei der Reinigungsprozess allerdings in Abhängigkeit von der persönlichen Vorgehensweise der Patienten, ihrem Alter und ihrem Erkrankungsgrad unterschiedlich konsequent durchgeführt wird. Darüber hinaus ist auch das Innere des Gehäuses des Inhalators, insbesondere von Arzneimittelrückständen, zu reinigen, da diese Rückstände zu Dosierungsproblemen führen können, wenn sie sich in unregelmäßigen Abständen lösen und mit der eigentlichen Dosis ausgebracht werden.

Es ist Aufgabe der Erfindung, einen Inhalator der eingangs genannten Art zu schaffen, der für einen Patienten einfach handhabbar ist.

Erfindungsgemäß wird die Aufgabe dadurch gelöst, das Gehäuse starr mit dem Mundstück verbunden ist.

Das Gehäuse bzw. der Innenraum des Gehäuses wird bereits herstellerseitig mit dem genau dosierten Arzneimittel bestückt und es ist nicht erforderlich, das Mundstück von dem Gehäuse abzuklappen, um eine mit dem Arzneimittel gefüllte Kapsel in das Gehäuse einzulegen, wie es aus dem Stand der Technik bekannt ist. Vielmehr ist das Mundstück derart starr mit dem Gehäuse verbunden, dass ein Benutzer des Inhalators das Gehäuse nicht zum Befüllen des Innenraums mit dem Arzneimittel öffnen kann. Dieser Inhalator ist insofern vorteilhaft, als er unter Verwendung einer geringen Anzahl von Einzelteilen kostengünstig zur einmaligen Verwendung herstellbar ist und lediglich absolut notwendige Bauteile, nämlich das Gehäuse und das Mundstück, umfasst. Durch die Ausgestaltung als Einmal-Inhalator ist auch dessen Handhabung vereinfacht, da insbesondere eine regelmäßige Reinigung entfällt und Arzneimittelrückstände in dem Inhalator die Anwendung nicht beeinflussen können. Das Mundstück kann ohne ergonomische Formgebung als einfaches Rohr ausgestaltet und mit dem Gehäuse beispielsweise verklipst sein. Im Weiteren ist es nicht erforderlich, dass der Benutzer bzw. Patient einen Inhalator und separat dazu die zu inhalierende Substanz mit sich führt.

Inhalatoren sind unter den Markennamen HandiHaler®, Spinhaler®, Rotahaler®, Aerolizer®, Flowcaps®, Turbospin®, AIR DPI®, Orbital®, Directhaler® bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler®, Rotahaler®, Aerolizer®, Inhalator®, HandiHaler®, Diskhaler®, Diskus®, Accuhaler®, Aerohaler®, Eclipse®, Turbohaler®, Turbuhaler®, Easyhaler®, Novolizer®, Clickhaler®, Pulvinal®, Novolizer®, SkyeHaler®, Xcelovair®, Pulvina®, Taifun®, MAGhaler®, Twisthaler® und der Jethaler® bekannt.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{ [2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino }ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 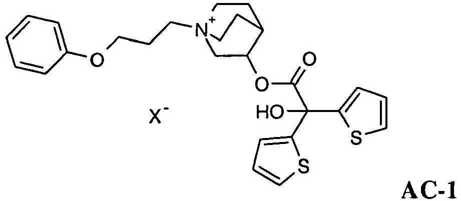 worin X ⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 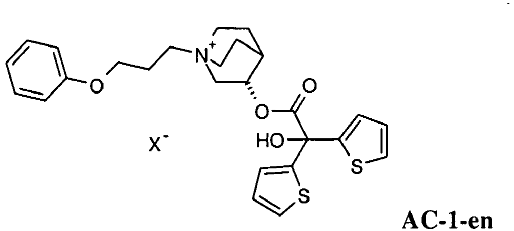 enthalten, worin X ⁻ die vorstehend genannten Bedeutungen aufweisen kann. Weiterhin bevorzugte Anticholinergika sind ausgewählt aus den Salzen der Formel **AC-2** 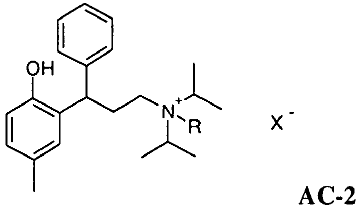 worin R entweder Methyl oder Ethyl bedeuten und worin X ⁻ die vorstehend genannte Bedeutungen aufweisen kann. In einer alternativen Ausführungsform kann die Verbindung der Formel **AC-2** auch in Form der freien Base **AC-2-base** vorliegen.

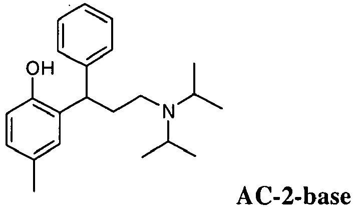

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-dinuoro-11β-hydroxy-16α-methyl-3-oxo-17α-(2,2,3,3-tertamethylcyctopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, A-riflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-l-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium-oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-1[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethy|amino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{ [4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxyl-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel mit den o.g. Wirkstoffen in Betracht, sowie deren Salze, Ester sowie die Kombination dieser Wirkstoffe, Salze und Ester.

Bevorzugt ist das Gehäuse unlösbar mit dem Mundstück gekoppelt. Wenn das Gehäuse z.B. mit dem Mundstück verklebt oder verschweißt ist, ist es dem Benutzer des Inhalators nicht möglich, an das Arzneimittel zu gelangen, ohne den Innhalator zu zerstören.

Zweckmäßigerweise weist das Gehäuse für die Inhalation pulverförmiger Arzneimittel eine Luftansaugöffnung auf. Beim Einatmen bzw. Inhalieren durch das Mundstück wird die durch die Luftansaugöffnung in den Innenraum gelangende Luft mit dem Arzneimittel beladen.

In Ausgestaltung ist dem Mundstück ein Bauteil zum Dispergieren von Partikeln zugeordnet. Das Bauteil sorgt für eine feine inhalierfähige Verteilung der Partikel des Arzneimittels. Zweckmäßigerweise ist das Bauteil einstückig mit dem Mundstück oder einem Inhalationskanal des Innenraums ausgebildet. Das Bauteil kann beispielsweise im Spritzgussverfahren gemeinsam mit dem aus Kunststoff gefertigten Mundstück bzw. dem Inhalationskanal des ebenfalls aus Kunststoff hergestellten Gehäuses gefertigt werden. In weiterer Ausgestaltung ist das Bauteil als Sieb oder dergleichen ausgeführt.

Vorzugsweise handelt es sich bei den Kunststoffen um Polymerisate, thermoplastische Polykondensate, Polyaddukte, abgewandelte Naturstoffe oder Kautschuke bzw. um Gemische dieser Kunststoffe.

Besonders bevorzugt sind hier Polyolefine, Vinylchlorid-Polymerisate, StyrolPolymerisate, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acryl-ester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden. Um auf eine zusätzliche Verpackung des Arzneimittels zu verzichten, ist zweckmäßigerweise das Arzneimittel in dem Innenraum aufgenommen. Nach einer weiteren Ausführungsform ist das Arzneimittel in einer in den Innenraum eingesetzten Kapsel aufgenommen. Die Kapsel zur Aufnahme des Arzneimittels hat sich insofern bewährt, als sie einen zusätzlichen Schutz vor Umgebungseinflüssen darstellt. Für eine weiterhin verbesserte Handhabung des Inhalators ist der Innenraum herstellerseitig mit dem Arzneimittel bzw. der das Arzneimittel aufnehmenden Kapsel gefüllt.

Vorzugsweise ist mindestens eine Nadel zum Einstechen der Kapsel vorgesehen. Insbesondere sind zwei Nadeln vorhanden, wobei die eine Nadel einem dem Mundstück zugewandten Bereich der Kapsel und die andere Nadel einem gegenüberliegenden Bereich zugeordnet ist.

In weiterer Ausgestaltung ist die Nadel mit einem Betätigungselement gekoppelt, das derart an einer Kapselkammer des Inhalators gelagert ist, dass mit seiner Beaufschlagung ein Einstechen der Nadel in die Kapsel und/oder ein Herausziehen der Nadel aus der Kapsel einhergeht. Das bei einer Beaufschlagung das Einstechen der Nadel in die Kapsel bewirkende Betätigungselement ist dann besonders einfach zu handhaben, wenn die Kapsel erst unmittelbar vor dem Gebrauch des Inhalators geöffnet werden soll. Das Betätigungselement kann entweder durch Druck, Zug oder Verschieben betätigbar sein. Zur Unterstützung des Herausziehens der Nadel aus der Kapsel ist das Betätigungselement gegenüber der Kapselkammer federbeaufschlagt. Erfindungsgemäß ist die Nadel im Auslieferungszustand des Inhalators in die Kapsel eingestochen. Befindet sich die Nadel im Auslieferungszustand des Inhalators bereits in der Kapsel, dann wird die Nadel durch die Beaufschlagung des Betätigungselementes entweder durch Druck oder Zug aus der Kapsel herausgezogen. Diese Vorgehensweise ist insofern vorteilhaft, als bei einem verhältnismäßig geringen Kraftaufwand seitens des Benutzers des Inhalators sichergestellt ist, dass die Nadeln ein Loch vorbestimmter Größe in die Kapsel eingebracht haben und damit die Ausbringungsrate des zu inhalierenden Arzneimittels sichergestellt ist. Im Weiteren fixieren die in die Kapsel ragenden Nadeln die Kapsel in einer vorbestimmten Lage im Innenraum des Gehäuses, also in einer Kapselkammer.

Vorzugsweise besteht die Nadel aus einem Kunststoff. Da der Inhalator lediglich einmal Verwendung findet und somit auch die Nadel, ist es nicht zwingend erforderlich, diese aus einem Edelstahl herzustellen. Selbstverständlich kann eine bekannte Nadel aus einem Metall, insbesondere einem Edelstahl, ebenfalls Verwendung finden. Der Fachmann wird in Abhängigkeit von den Anforderungen an die Nadel den entsprechenden Werkstoff auswählen und deren Geometrie bestimmen.

Alternativ ist die Kapsel mit zwei zueinander beabstandeten Öffnungen auf entsprechenden Haltern in der Kapselkammer lösbar angeordnet ist. Sonach ist es nicht erforderlich, bewegbare Nadeln zu lagern. Vielmehr kann der Benutzer des Inhalators die Kapsel beispielsweise durch eine ruckartige Bewegung des Inhalators von den z.B. als Stifte ausgebildeten Haltern lösen.

Damit der Inhalator ohne weiteres Zutun seitens des Benutzers einsatzbereit ist, ist bevorzugt die Kapsel derart auf den Haltern befestigt, dass sie sich aufgrund eines durch das Inhalieren verursachten Luftstroms löst und die Substanz freigibt.

Zur optischen Kontrolle ist die Kapselkammer aus einem transparenten Werkstoff gefertigt. Da die Kapselkammer nicht von weiteren Bauteilen umgeben ist, kann der Benutzer unmittelbar kontrollieren, ob beispielsweise das gesamte vorhandene Arzneimittel inhaliert wurde.

Um das zu inhalierende Arzneimittel und den Inhalator vor Umgebungseinflüssen zu schützen, ist der Inhalator mit einer luftdichten Umverpackung, insbesondere einem Folienbehälter, versehen. Eine derartige Umverpackung ist handelsüblich. Alternativ oder zusätzlich sind das Mundstück und/oder die Luftansaugöffnung mit einer abnehmbaren Kappe dicht verschlossen. Aufgrund dieser Maßnahmen ist der Innenraum des Inhalators mit dem Arzneimittel mit einem minimalen Verpackungsaufwand vor insbesondere das Arzneimittel schädigenden Einflüssen, wie beispielsweise Feuchtigkeit, geschützt. Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand mehrerer Ausführungsbeispiele unter Bezugnahme auf die zugehörigen Zeichnungen näher erläutert. Es zeigt:
- Fig.1: eine schematische Darstellung eines nicht erfindungsgemäßen Inhalators,
- Fig.2: eine schematische Darstellung des Inhalators nach Fig. 1 in einer alternativen erfindungsgemäßen Ausgestaltung und
- Fig.3: eine schematische Darstellung des Inhalators nach Fig. 1 in einer weiteren alternativen erfindungsgemäßen Ausgestaltung.

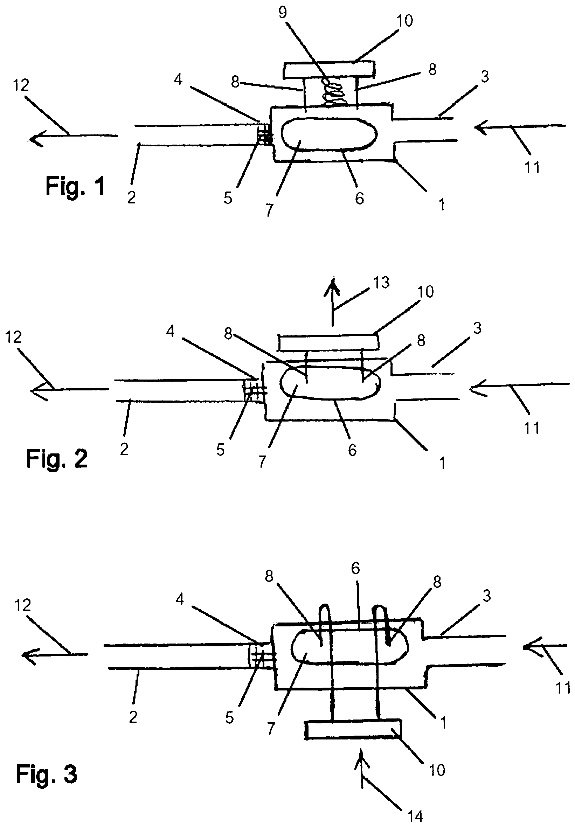

Der Inhalator gemäß Fig. 1 besteht im Wesentlichen aus einem, einen Innenraum zur Aufnahme eines inhalationsfähigen Arzneimittels umfassenden Gehäuse 1, das einerseits ein Mundstück 2 und andererseits eine Lufteinlassöffnung 3 aufweist. In einem Inhalationskanal 4 ist auf der Seite des Mundstücks 2 ein Sieb 5 zum Dispergieren von Partikeln des zu inhalierenden Arzneimittels angeordnet. In eine Kapselkammer 6 im Innenraum des Gehäuses 1 ist eine ein pulverförmiges Arzneimittel enthaltende Kapsel 7 eingesetzt, die durch zwei beabstandet zueinander in dem Gehäuse 1 verschiebbar gelagerte Nadeln 8 zu öffnen ist. Zum Herausbewegen der Nadeln 8 aus der Kapsel 7 ist eine Druckfeder 9 vorgesehen, die sich zum einen an dem Gehäuse 1 und zum anderen an einem mit den Nadeln 8 verbundenen Betätigungselement 10 abstützt. Die Kapsel 7 wird herstellerseitig in die Kapselkammer 6 des Gehäuses 1 eingelegt und das Gehäuse 1 anschließend fest mit dem vorliegend rohrförmig gestalteten Mundstück 2 verbunden, wonach eine Entnahme der Kapsel 7 unmöglich ist.

Ein Benutzer des Inhalators entnimmt diesen einer luftdicht geschlossenen Verpackung, die insbesondere das Arzneimittel vor Umgebungseinflüssen schützt, und beaufschlagt anschließend das Betätigungselement 10 um die beiden Nadeln 8 in die Kapsel 7 einzustechen. Nach dem Loslassen des Betätigungselementes 10 kehrt dieses aufgrund der Wirkung der Druckfeder 9 in seine Ausgangslage zurück. Der Benutzer nimmt zur Inhalation das Mundstück 2 in den Mund und saugt durch die Lufteinlassöffnung 3 gemäß dem Pfeil 11 Luft in das Gehäuse 1, die die Kapsel 7 in Vibration versetzt, wodurch das Arzneimittel ausgetragen wird und durch das Mundstück 2 gemäß dem Pfeil 12 in die Atemwege des Benutzers gelangt. Nach der Inhalation kann der Benutzer den Inhalator entsorgen, da dieser zur Einmalverwendung bestimmt ist.

Nach Fig. 2 sind die Nadeln 8 bereits herstellerseitig in die Kapsel 7 eingestochen, weshalb diese zur Benutzung des Inhalators mittels des Betätigungselementes 10 entsprechend dem Pfeil 13 aus der Kapsel 7 herausgezogen werden müssen, um die Öffnungen in der Kapsel 7 zum Austritt des Arzneimittels freizugeben. Demzufolge ist das Einstechen der Kapsel 7 durch den Patienten nicht erforderlich und der Durchmesser der Nadeln 8 kann an die erforderliche Ausbringungsrate des Inhalationspulvers ohne Berücksichtigung der Betätigungskraft beim Einstechen der Nadeln 7 in die beispielsweise aus einem Kunststoff gefertigte Kapsel 7 angepasst werden. Die in die Kapsel 7 eingestochenen Nadeln 7 verschließen die Kapsel 7 aufgrund einer elastischen Rückstellung des Materials, aus dem die Kapsel 7 gefertigt ist, weshalb das pulverförmige Arzneimittel nicht aus der Kapsel 7 austreten kann, und fixieren die Kapsel 7 in der Kapselkammer 6.

Bei dem Inhalator gemäß Fig. 3 sind die Nadeln 8 ebenfalls bereits herstellerseitig in die Kapsel 7 eingestochen. Das Betätigungselement 10 ist derart an dem Gehäuse 1 gelagert, dass es zum Entfernen der Nadeln 8 aus der Kapsel 7 zur Freigabe der Öffnungen in der Kapsel 7 entsprechend dem Pfeil 14 durch Druck zu beaufschlagen ist.

## Patentansprüche

1. Inhalator zur Verabreichung von einem Arzneimittel in Form von inhalationsfähigen Substanzen, Substanzformulierungen oder -mischungen mit einem einen Innenraum zur Aufnahme des Arzneimittels aufweisenden Gehäuse (1), das mit einem Mundstück (2) gekoppelt ist, wobei das Gehäuse (1) starr mit dem Mundstück (2) verbunden ist, und wobei das Arzneimittel in einer in den Innenraum eingesetzten Kapsel (7) aufgenommen ist, und
wobei mindestens eine Nadel (8) zum Einstechen der Kapsel (7) vorgesehen ist, **dadurch gekennzeichnet, dass** der Innenraum herstellerseitig mit der Kapsel (7) gefüllt ist und die Nadel (8) bereits herstellerseitig in die Kapsel (7) eingestochen ist.

2. Inhalator nach Anspruch 1, **dadurch gekennzeichnet, dass** das Gehäuse (1) unlösbar mit dem Mundstück (2) gekoppelt ist.

3. Inhalator nach Anspruch 1 oder 2, **dadurch gekennzeichnet, dass** das Gehäuse (1) für die Inhalation pulverförmiger Arzneimittel eine Luftansaugöffnung (3) aufweist.

4. Inhalator nach einem der Ansprüche 1 bis 3, **dadurch gekennzeichnet, dass** dem Mundstück (2) ein Bauteil zum Dispergieren von Partikeln zugeordnet ist.

5. Inhalator nach Anspruch 4, **dadurch gekennzeichnet, dass** das Bauteil einstückig mit dem Mundstück (2) oder einem Inhalationskanal (4) des Innenraums ausgebildet ist.

6. Inhalator nach einem der Ansprüche 4 oder 5, **dadurch gekennzeichnet, dass** das Bauteil als Sieb (5) oder dergleichen ausgeführt ist.

7. Inhalator nach einem der Ansprüche 1 bis 6, **dadurch gekennzeichnet, dass** die Nadel (8) mit einem Betätigungselement (10) gekoppelt ist, das derart an einer Kapselkammer (6) des Inhalators gelagert ist, dass mit seiner Beaufschlagung ein Herausziehen der Nadel (8) aus der Kapsel (7) einhergeht.

8. Inhalator nach Anspruch 7, **dadurch gekennzeichnet, dass** das Betätigungselement (10) gegenüber der Kapselkammer (6) federbeaufschlagt ist.

9. Inhalator nach einem der Ansprüche 1 bis 8, **gekennzeichnet dadurch, dass** er zur einmaligen Verwendung geeignet ist.

## Claims

1. Inhaler for administering a medicament in the form of inhalable substances, formulations or mixtures of substances, with a housing (1) comprising an inner cavity for holding the medicament, which is coupled to a mouthpiece (2), the housing (1) being rigidly connected to the mouthpiece (2), and the medicament being held in a capsule (7) inserted in the inner cavity, and
at least one pin (8) being provided for piercing the capsule (7), **characterised in that** the inner cavity is filled with the capsule (7) by the manufacturer, and the pin (8) has already pierced the capsule (7) in a process carried out by the manufacturer.

2. Inhaler according to claim 1, **characterised in that** the housing (1) is non-releasably coupled to the mouthpiece (2).

3. Inhaler according to either claim 1 or claim 2, **characterised in that** the housing (1) has an air-intake opening (3) for the inhalation of powdered medicaments.

4. Inhaler according to any of claims 1 to 3, **characterised in that** a component for dispersing particles is associated with the mouthpiece (2).

5. Inhaler according to claim 4, **characterised in that** the component is formed in one piece with the mouthpiece (2) or an inhalation channel (4) of the inner cavity.

6. Inhaler according to either claim 4 or claim 5, **characterised in that** the component is constructed as a screen (5) or the like.

7. Inhaler according to any of claims 1 to 6, **characterised in that** the pin (8) is coupled to an actuating element (10) which is mounted on a capsule chamber (6) of the inhaler in such a way that actuation thereof is accompanied by the pin (8) being pulled out of the capsule (7).

8. Inhaler according to claim 7, **characterised in that** the actuating element (10) is springloaded relative to the capsule chamber (6).

9. Inhaler according to any of claims 1 to 8, **characterised in that** it is suitable for one-time use.

## Revendications

1. Inhalateur pour l'administration d'un médicament sous forme de substances, de formulations de substances ou de mélanges de substances inhalables avec un boîtier (1) présentant un espace intérieur pour la réception du médicament, qui est couplé avec un embout (2), dans lequel le boîtier (1) est relié rigidement à l'embout (2), et dans lequel le médicament est reçu dans une capsule (7) insérée dans l'espace intérieur, et
dans lequel au moins une aiguille (8) est prévue pour le perçage de la capsule (7),
**caractérisé en ce que** l'espace intérieur est rempli côté fabricant avec la capsule (7) et l'aiguille (8) est déjà percée côté fabricant dans la capsule (7).

2. Inhalateur selon la revendication 1, **caractérisé en ce que** le boîtier (1) est couplé de manière inamovible avec l'embout (2).

3. Inhalateur selon la revendication 1 ou 2, **caractérisé en ce que** le boîtier (1) présente une ouverture d'aspiration d'air (3) pour l'inhalation de médicaments pulvérulents.

4. Inhalateur selon l'une quelconque des revendications 1 à 3, **caractérisé en ce qu'**un composant de dispersion de particules est associé à l'embout (2).

5. Inhalateur selon la revendication 4, **caractérisé en ce que** le composant est réalisé d'un seul tenant avec l'embout (2) ou avec un canal d'inhalation (4) de l'espace intérieur.

6. Inhalateur selon l'une quelconque des revendications 4 ou 5, **caractérisé en ce que** le composant est réalisé en tant que tamis (5) ou similaire.

7. Inhalateur selon l'une quelconque des revendications 1 à 6, **caractérisé en ce que** l'aiguille (8) est couplée avec un élément d'actionnement (10), qui est logé au niveau d'une chambre de capsule (6) de l'inhalateur de telle sorte qu'un retrait de l'aiguille (8) de la capsule (7) va de pair avec sa sollicitation.

8. Inhalateur selon la revendication 7, **caractérisé en ce que** l'élément d'actionnement (10) est sollicité par ressort par rapport à la chambre de capsule (6).

9. Inhalateur selon l'une quelconque des revendications 1 à 8, **caractérisé en ce qu'**il ne convient que pour un usage unique.
